# EUROPEAN PATENT APPLICATION

(11) **EP 2 306 199 A1**
(43) Date of publication of application: **06.04.2011**
(21) Application number: 09252309.1
(22) Date of filing: 29.09.2009
(51) Int. Cl.: G01N 33/66, G01N 33/58, G01N 30/06

(54) **Reductive amination and analysis of carbohydrates using 2-picoline borane as reducing agent**

(71) Applicant: Academisch Ziekenhuis Leiden Acting Under The Name Leiden University Medical Center, 2300 RC Leiden (NL)
(72) Inventor: Ruhaak, Lucia Renee, 2341 VX Oegstgeest (NL); Wuhrer, Manfred, 2252 BR Voorschoten (NL)
(74) Representative: Pugh, Robert Ian

(57) **Abstract**

The application provides the use of 2-picoline borane (2-PB) for the reductive amination of carbohydrates, especially glycans from blood plasma, and describes a process for labelling a carbohydrate, the process comprising contacting the carbohydrate with a labelling agent, eg. 2-aminobenzoic acid, 2-aminobenzamide, APTS etc. in the presence of 2-picoline borane as reducing agent. Labelling the carbohydrate in this way facilitates detection using HPLC techniques, eg. HILIC-HPLC-FL, HILIC-ESI-IT-MS, or capillary electrophoresis.

## Description

The invention relates to the reductive amination of carbohydrates.

Protein-linked glycans are involved in important biological processes such as cell-cell interaction, receptor activation and molecular trafficking (Ohtsubo et al, Cell 2006, 126, 855-67). Recent research into the role of glycans in drug therapy and the search for glycans as biomarkers in specific diseases has created the need for fast and sensitive high-throughput glycoanalytical methods (Packer et al, Proteomics 2008, 8, 8-20; and Wuhrer, M. Expert.Rev.Proteomics 2007, 4, 135-36).

Such methods comprise HPLC (Royle et al, Anal.Biochem. 2008, 376, 1-12; and Ruhaak et al, Anal.Chem. 2008, 80, 6119-26) and capillary electrophoresis (CE) (Kamoda et al, J.Chromatogr.A 2006, 1133, 332-39) coupled to UV, fluorescence or mass spectrometric detection. Alternatively, mass spectrometry may be used as a stand-alone technique (Qian et al, Anal.Biochem. 2007, 364, 8-18; and Jang-Lee et al, Methods Enzymol. 2006, 415, 59-86). Many approaches involve glycan derivatization for the introduction of a UV-absorbing or fluorescent tag (Anumula, K. R. Anal.Biochem. 2006, 350, 1-23; and Shilova et al, Bioorg.Khim. 2003, 29, 339-55).

The listing or discussion of a prior-published document or any background in the specification should not necessarily be taken as an acknowledgement that a document or background is part of the state of the art or is common general knowledge.

A widely used labelling technique involves coupling of an oligosaccharide to an amine-substituted chromophore or fluorophore by reductive amination. In a reversible reaction, the open-ring form of the carbohydrate reacts with the amine group and eliminates water to form a Schiff base. In a second, irreversible reaction, the Schiff base is reduced to form a secondary amine. The reducing agent that is most widely used in this reaction is sodium cyanoborohydride (NaBH₃CN). A major drawback of this reagent is that upon hydrolysis it readily forms the toxic, volatile compound hydrogen cyanide. A further drawback of this reagent is that it is believed to be too strong a reducing agent, resulting in at least some direct reduction of the oligosaccharide rather than reductive amination.

An alternative reducing agent sodium triacetoxyborohydride (NaBH(OAc)₃) has been introduced for the reductive amination of carbohydrates with 4-amino-N-[2-(diethylamino)ethyl] benzamide (DEAEAB), and this approach was hypothesized to be suitable for all amine-labels (Dalpathado et al, Anal.Bioanal.Chem. 2005, 381, 1130-37). This reducing agent, however, is not broadly applied in glycan analysis. This is believed to be because it is not reactive enough.

The subject invention addresses the above and other deficiencies by providing the use of 2-picoline borane (2-PB) for the reductive amination of a carbohydrate. Surprisingly, the inventors have found that 2-PB is particularly effective and useful as a reductive amination reagent for the labelling of carbohydrates. Thus, the invention also provides a process for labelling a carbohydrate, the process comprising contacting the carbohydrate with a labelling agent in the presence of 2-PB to produce a labelled carbohydrate.

Without being bound by theory, it is believed that the use of 2-PB for the reductive amination (e.g. labelling) of carbohydrates has the following unexpected advantages:
(i) Comparable, if not improved, conversion and/or selectivity for reductive amination of carbohydrates compared to NaBH₃CN; and/or
(ii) Improved conversion and selectivity for reductive amination of carbohydrates compared to NaBH(OAc)₃; and/or
(iii) Reduced toxicity compared to NaBH₃CN.

In a preferred embodiment, the carbohydrate is reacted with a labelling agent in the presence of 2-PB. Any suitable labelling agent may be used in the invention. Typically, the labelling agent will include an amine group which can react with the open-ring form of the reducing end of a carbohydrate. Examples include amine-substituted chromophores or fluorophores and other amine containing labelling agents such as alanine, and [¹³C₆]-alanine. Amine-substituted chromophores or fluorophores are currently preferred labelling agents.

Examples of suitable amine-substituted chromophores or fluorophores include 2-aminobenzoic acid (2-AA), 2-aminobenzamide (2-AB), 8-aminopyrene-1,3,6-trisulfonic acid (APTS), 2-aminopyridine (2-AP), [D₆]-AP, and [D₄]-2-AA. The amine-substituted chromophores or fluorophores may carry a further reactive functional group. Examples of such multifunctional tags include 2,5-diaminopyridine and 2-amino-6-amidobiotinylpyridine (BAP). 2-AA, 2-AB and APTS currently are preferred amine-substituted chromophores or fluorophores (e.g. APTS). Other suitable amine-substituted chromophores or fluorophores will be apparent to the skilled person, for example from Anumula, K. R. Anal.Biochem. 2006, 350, 1-23, which is incorporated herein by reference.

By the term "a carbohydrate", we include one or more monosaccharides, oligosaccharides and polysaccharides, typically containing from 1 to 40 component sugars, or a mixture of such saccharides.

Suitable carbohydrates may be derived from human, animal or plant origin, or from recombinantly expressed or biotechnologically expressed proteins.

Suitable synthetic methods for preparing carbohydrates include organic synthesis, and full or partial hydrolysis or degradation of polysaccharides. Examples include oligosaccharides (also referred to herein as glucose ladders) that have been prepared by partial acid hydrolysis of dextran. Other methods to synthesize oligosaccharides will be known to the skilled person (see for example Seeberger, P.H., Carbohydrate Research 2008, 343, 1889-1896, and Seeberger, P.H., Chem Soc Rev. 2008, 37, 19-28. which are incorporated herein by reference).

Glycans (such as N-glycans), including those derived from glycoproteins and glycolipids, are a further example of carbohydrates that can be used in the subject invention, such as N-glycans prepared from human plasma.

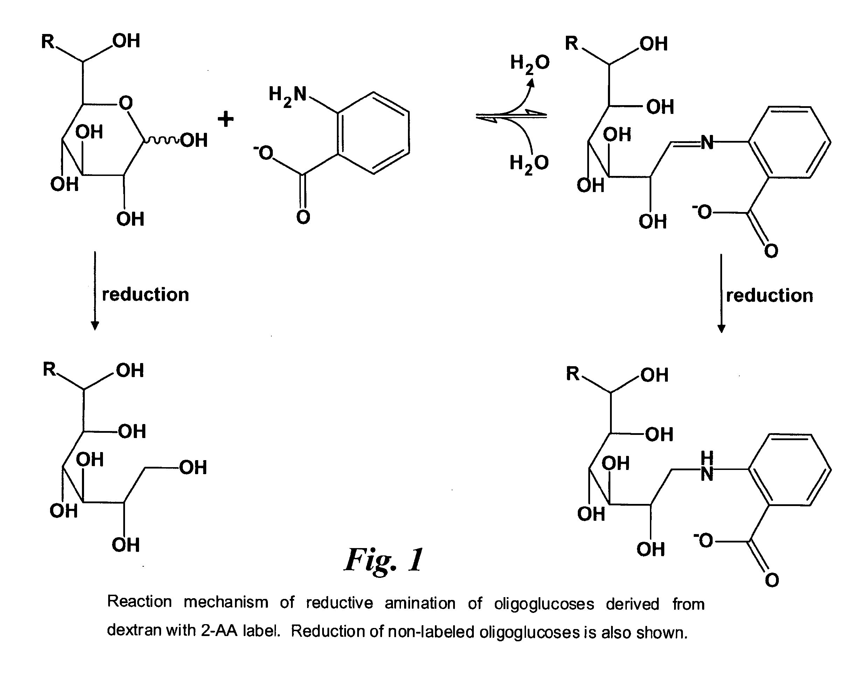

Figure 1 depicts the reaction mechanism of reductive amination of oligoglucoses derived from dextran with a 2-AA label. Direct reduction of non-labelled oligoglucoses is also shown.

As shown in Figure 1, the reductive amination proceeds in two steps: a first (reversible) reaction in which the carbohydrate reacts with the primary amine to form a Schiff base, followed by a second (irreversible) reaction in which the Schiff base is reduced to form the secondary amine. The invention can be carried out in two discreet steps corresponding to these two reactions. Preferably, however, both reactions are carried out in one-pot. This has the advantage of speeding up the process and reducing the handling time.

The use of 2-PB in the reductive amination (e.g. labelling) of carbohydrates is thought to lead to greater selectivity to the desired amine product (*via* the Schiff base imine) over the undesired direct reduction product of the carbohydrates, compared to existing reductive amination agents such as NaBH₃CN and/or NaBH(OAc)₃.

2-PB is also believed to result in greater conversion of carbohydrates to the desired amine product (*via* the Schiff base imine) compared to existing reductive amination agents such as NaBH₃CN and/or NaBH(OAc)₃.

As a consequence of the improved activity and/or selectivity of 2-PB in the reductive amination (e.g. labelling) of carbohydrates compared to known reagents such as NaBH(OAc)₃ and NaBH₃CN, 2-PB may be used in reduced quantities compared to those reagents. This further reduces the toxicity (and health risk to researchers) and environmental impact of carbohydrate reductive amination (e.g. labelling), over and above the inherent reduced toxicity of 2-PB compared to NaBH₃CN.

In one embodiment, the concentration of 2-PB in the process of the invention is less than the concentration of NaBH(OAc)₃ or NaBH₃CN (preferably NaBH₃CN) required to obtained comparable conversion of the carbohydrate. For example, 2-PB may be used in less than 90%, such as less than 70%, preferably less than 50%, such as less than 30%, in molar quantities, compared to NaBH(OAc)₃ or NaBH₃CN (preferably NaBH₃CN) in order to attain essentially the same conversion of carbohydrate.

A further advantage of 2-PB in the reductive amination (e.g. labelling) of carbohydrates compared to known reagents such as NaBH(OAc)₃ and NaBH₃CN, is that 2-PB can lead to more robust labelling profiles (see Example 2, for instance). Without being bound by theory, it is believed that 2-PB exhibits reduced dependence of labelling efficacy on structural features such as charge, compared to known reagents such as NaBH(OAc)₃ and NaBH₃CN.

The invention allows carbohydrates to be easily and safely detected in a sample. Accordingly, the invention provides a method for detecting a carbohydrate in a sample, the method comprising:
(i) contacting the carbohydrate in the sample with a labelling agent in the presence of 2-picoline borane (2-PB) to produce a labelled carbohydrate as defined herein; and
(ii) detecting the labelled carbohydrate.

Any suitable detecting method may be used for step (ii), such as liquid chromatography coupled to fluorescence detection, UV absorbance detection, and/or detection by mass spectrometry. A preferred method is hydrophilic interaction chromatography with fluorescence detection (HILIC-HPLC-FL).

The ease, efficacy, reduced toxicity and/or reduced cost of the invention (compared to the prior art) means that it applicable to high-throughput analysis. Thus, the invention may be carried out simultaneously on a plurality of samples potentially containing a carbohydrate. For example, the method for detecting a carbohydrate described above may be applied to a plurality of samples simultaneously.

The reaction of the carbohydrate with labelling agent and 2-PB may be carried out at any suitable reaction temperature for any suitable length of time. Typical reaction times are from about 1 minute to about 10 hours, such as from about 5 minutes to about 5 hours. Typical reaction temperatures are from about 0 to abut 100 °C, such as from about 20 to about 80 °C. Preferably, the reaction is carried out in one-pot.

The reaction may be carried out in any suitable solvent, including water, DMSO, (glacial) acetic acid, acetonitrile, ethanol, or mixtures of one or more of the foregoing. The reaction may be carried out under aqueous or water-free conditions.

In a preferred embodiment, an acid is used to aid the reductive amination. Any suitable acid may be used, such as an organic acid (e.g. acetic acid). Without being bound by theory, it is believed that the acid increases the efficacy of the reductive amination.

The invention will now be illustrated by the following non-limiting examples.

### Example 1: labelling of glucose ladder and N-glycans from human plasma with 2-AA and 2-AB using NaBH₃CN, NaBH(OAc)₃ and 2-PB

### Materials

Dimethylsulphoxide (DMSO), ammonium hydroxide, formic acid, Nonidet P-40 (NP-40), 2-aminobenzoic acid (2-AA), 2-aminobenzamide (2-AB), NaBH₃CN, NaBH(OAc)₃ and 2-picolineborane (2-PB) were obtained from Sigma-Aldrich (Zwijndrecht, The Netherlands). Sodium dodecyl sulphate (SDS) was bought from United States Biochemicals (Cleveland, OH). PNGaseF was obtained from Roche Diagnostics (Mannheim, Germany). Glacial acetic acid was purchased from Merck (Darmstadt, Germany). Acetonitrile was purchased from Biosolve (Valkenswaard, The Netherlands). Dextran 10.000 was obtained from Pharmacia/GE Healthcare (Uppsala, Sweden).

### Preparation of oligosaccharides

A glucose ladder was produced by partial acid hydrolysis of 10 mg of dextran 10.000 in 1 ml of 1 M TFA (1 h at 80°C). The sample was subsequently diluted using 4 ml of water. N-glycans from human plasma were prepared as described in Ruhaak et al, Anal.Chem. 2008, 80, 6119-26, which is incorporated herein by reference. In summary, proteins from 10 µl of plasma were denatured after addition of 20 µl 2% SDS by incubation at 60°C for 10 min. Subsequently, 10 µl 4% NP-40 and 0.5 mU of PNGase F in 10 µl 5x PBS was added to the samples. The samples were incubated overnight at 37°C for N-glycan release.

### Labelling of oligosaccharides

50 µl of oligosaccharide solutions (either glucose-ladder or plasma N-glycans without prior purification) were mixed with 25 µl of a freshly prepared solution of label (2-AA or 2-AB; 48 mg/ml in DMSO containing 15% glacial acetic acid). 25 µl aliquots of freshly prepared reducing agent solutions (1 M NaBH₃CN, 2-picoline borane, or NaBH(OAc)₃ in DMSO) were added, followed by 5 min of shaking and incubation at 65 °C for 2 hours.

To allow comparison to other studies, 2-AB labelling was also performed under water-free conditions, as described in Bigge et al, Anal.Biochem. 1995, 230, 229-38, which is incorporated herein by reference. In summary, 50 µl of glucose-ladder samples were brought to dryness using vacuum centrifugation. Oligosaccharide samples were then mixed with 25 µl of a freshly prepared solutions of 96 mg/ml 2-AB in DMSO containing 15% glacial acetic acid. 25 µl aliquots of freshly prepared reducing agent solution (2M NaBH₃CN, 2-picoline borane, or NaBH(OAc)₃ in DMSO) were added, followed by 5 min of shaking and incubation at 65 °C for 2 hours.

The reaction mixture was allowed to cool down to room temperature. Samples were diluted 1:3(v/v) with acetonitrile prior to analysis by hydrophilic interaction chromatography with fluorescence detection (HILIC-HPLC-FL).

### Purification by graphitised carbon solid phase extraction (PGC-SPE)

Free label and reducing agent were removed from the samples using porous graphitic carbon SPE. The carbograph SPE cartridges (Grace, Breda, The Netherlands) were conditioned with 2 ml of 80% acetonitrile in water, followed by equilibration with 3 ml of water. Samples were mixed with 300 µl of water and were loaded on the cartridges. After washing with 4 ml water, oligosaccharides were eluted using 1ml of 50% acetonitrile containing 0.1 % TFA. Eluates were diluted 1:1 (v/v) with acetonitrile prior to analysis by hydrophilic interaction chromatography coupled to electrospray ionization with ion trap MS detection (HILIC-ESI-IT-MS(/MS)).

### HILIC-HPLC-FL

Labeled N-glycans were analyzed using HILIC-HPLC with fluorescence detection. The Ultimate LC system (Dionex, Sunnyvale, CA) consisted of a Famos autosampler, a Switchos module with a loading pump, and an Ultimate pump module. The system was connected to a fluorescence detector (FP-2020 plus; Jasco, Easton, MD), which was operated at excitation wavelength 360 nm and emission wavelength 420 nm. The system was controlled by Chromeleon software and equipped with a 2.0 mm x 10 mm TSK gel-Amide 80 trapping column and a 2.0 mm x 250 mm TSK gel-Amide 80 analytical column (Tosoh Biosciences, Stuttgart, Germany).

Sample-containing wells in a 800 µl, v-bottom polypropylene 96-well plate (Westburg, Leusden, The Netherlands) were sealed with silicon lids (Labservices, Breda, The Netherlands), and placed in the autosampler. A 20 µl aliquot was injected using a full loop injection procedure. The labelled oligosaccharides were transferred to the trapping column and washed using acetonitrile:50 mM ammonium formate (pH 4.4; 80:20, by volume) for 3 min at a flow rate of 150 µl/min. Subsequently the trapping column was switched in line with the analytical column which was equilibrated with 70% acetonitrile (solvent A), 30% ammonium formate (50 mM, pH 4.4 (solvent B)) at a flow rate of 150 µl/min. A linear gradient was applied with Solvent B increasing from 30% (0 min) to 60% (87 min) followed by 5 min isocratic elution at 60% solvent B and re-equilibration of the column at 30% solvent B for 15 min.

### HILIC-ESI-IT-MS(/MS)

HILIC-nanoLC-ESI-ion trap (IT)-MS/MS of was performed on a Amide-80 column (3µm particles; 75 µm x 150 mm; Tosoh Biosciences) using an Ultimate 3000 nanoLC system (Dionex) equipped with a guard column (5 µm Amide-80 170 µm x 10 mm). Samples were brought to an acetonitrile content of 75%, and 10 µl samples were transferred to the guard column, which was washed for 5 min with acetonitrile:50 mM ammonium formate (pH 4.4, 90:10, v/v). The guard column was then brought in line with the nano column which was operated at a flow rate of 400 nl/min and equilibrated with 17.5% solvent A (50 mM ammonium formate, pH 4.4) and 82.5% solvent B (acetonitrile:50 mM ammonium formate, pH 4.4, 80:20, v/v). After an immediate step to 25% solvent A, a linear gradient was applied to 40% solvent A within 30 min, followed by a 4 min wash at 100 % solvent A and re-equilibration at 17.5% solvent A for another 25 min. The nanoLC system was directly coupled to an Esquire High Capacity Trap (HCTultra) ESI-IT-MS (Bruker Daltonics) equipped with an online nano-spray source operating in the positive-ion mode.

For electrospray (900-1200 V), capillaries (360 µm OD, 20 µm ID with 10 µm opening) from New Objective (Cambridge, MA, USA) were used. The solvent was evaporated at 165°C with a nitrogen stream of 6 I/min. Ions from m/z 400 to m/z 2000 were registered. Automatic fragment ion analysis was enabled for precursors between m/z 1170 and m/z 1180, resulting in tandem mass spectra of the reduced Glc₇-species.

### Data processing

For the HILIC-HPLC-FL data, peak intensities in mV were determined for five peaks from the glucose standard. Results from duplicate analyses were averaged. The differences between replicates were less than 10%. The HILIC-ESI-IT-MS data were analyzed using Data analysis version 4.0 software (Bruker Daltonics). Peak intensities of proton, ammonium, sodium and potassium ions from Glc₄ to Glc₈ were determined for both labelled and non-labelled species. Both single and double charged ions were considered. Intensities of all adducts were summed, and the relative abundances of labelled versus non-labelled glycans were calculated.

### Results

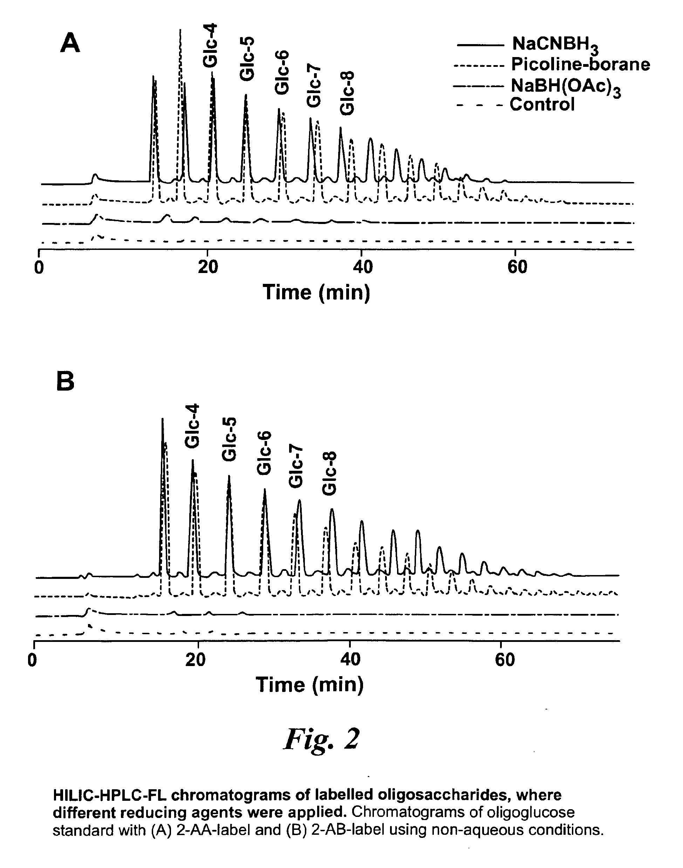

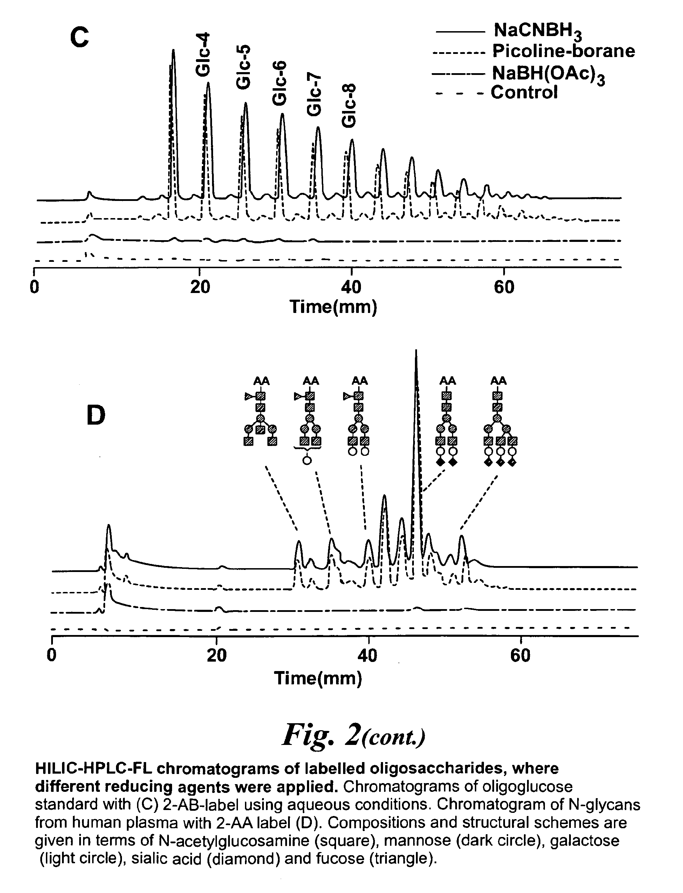

The reducing agents sodium triacetoxyborohydride, 2-picoline-borane and sodium cyanoborohydride were compared in the labelling of N-glycans by reductive amination. Dextran-derived oligoglucoses and N-glycans from human plasma were labelled using the aforementioned three reducing agents. Oligoglucoses were labelled with both the amines 2-AA and 2-AB, while plasma-derived N-glycans were labeled with 2-AA only. All samples were analyzed using HILIC-HPLC-FL. Oligoglucose ladders were additionally purified by graphitized carbon-SPE and analyzed using HILIC-LC-ESI-IT-MS. HILIC-HPLC-FL chromatograms of the glucose ladder after labelling using different reducing agents and labels are depicted in Figure 2 A, B and C. From these data, average peak heights of the Glc₄ to Glc₈ oligomers were determined (see Table 1 below).

**Table 1: Intensities of dextran derived oligoglucose compounds labelled with the aid of different reducing agents and their labelling efficiency. Intensities are shown in mV. n.d. = not determined**

| ***Reducing agent*** | ***Oligoglucose*** | *2-AA* | *2-AB,* | *2-AB,* |
|---|---|---|---|---|
| | | | *non- aqueous* | *aqueous* |
| **sodium cyanoborohydride** | Glc₄ | 598 (100%) | 585 (96%) | 568 (n.d.) |
| | Glc₅ | 501 (100%) | 476 (97%) | 463 (n.d.) |
| | Glc₆ | 433 (100%) | 427 (98%) | 404 (n.d.) |
| | Glc₇ | 374 (86%) | 352 (98%) | 342 (n.d.) |
| | Glc₈ | 313 (n.d) | 310 (n.d.) | 287 (n.d.) |
| **2-picoline-borane** | Glc₄ | 789 (100%) | 594 (99%) | 601 (n.d.) |
| | Glc₅ | 605 (100%) | 492 (98%) | 501 (n.d.) |
| | Glc₆ | 486 (100%) | 399 (98%) | 428 (n.d.) |
| | Glc₇ | 476 (90%) | 385 (98%) | 364 (n.d.) |
| | Glc₈ | 376 (n.d.) | 328 (n.d.) | 317 (n.d.) |
| **sodium triacetoxyborohydride** | Glc₄ | 22 (0%) | 13 (4%) | 7 (n.d.) |
| | Glc₅ | 18 (4%) | 10 (6%) | 6 (n.d.) |
| | Glc₆ | 16 (1 %) | 7 (6%) | 5 (n.d.) |
| | Glc₇ | 13 (1%) | 5 (4%) | 4 (n.d.) |
| | Glc₈ | 10 (n.d.) | 2 (n.d.) | 3 (n.d.) |
| **Control (no reducing agent)** | Glc₄ | 0 (0%) | 0 (0%) | 0 (n.d.) |
| | Glc₅ | 0 (0%) | 0 (0%) | 0 (n.d.) |
| | Glc₆ | 0 (0%) | 0 (0%) | 0 (n.d.) |
| | Glc₇ | 0 (0%) | 0 (0%) | 0 (n.d.) |
| | Glc₈ | 0 (n.d.) | 0 (n.d.) | 0 (n.d.) |

The observed intensities for fluorescently labelled glucose oligomers were very similar for 2-picoline-borane and NaBH₃CN, while the use of NaBH(OAc)₃ resulted in much lower intensities. This holds true for 2-AA labelling as well as 2-AB labelling under both aqueous and non-aqueous conditions. The labelling of oligoglucoses with 2-AB was just as efficient under aqueous as under non-aqueous conditions, for both sodium cyanoborohydride and 2-picoline-borane.

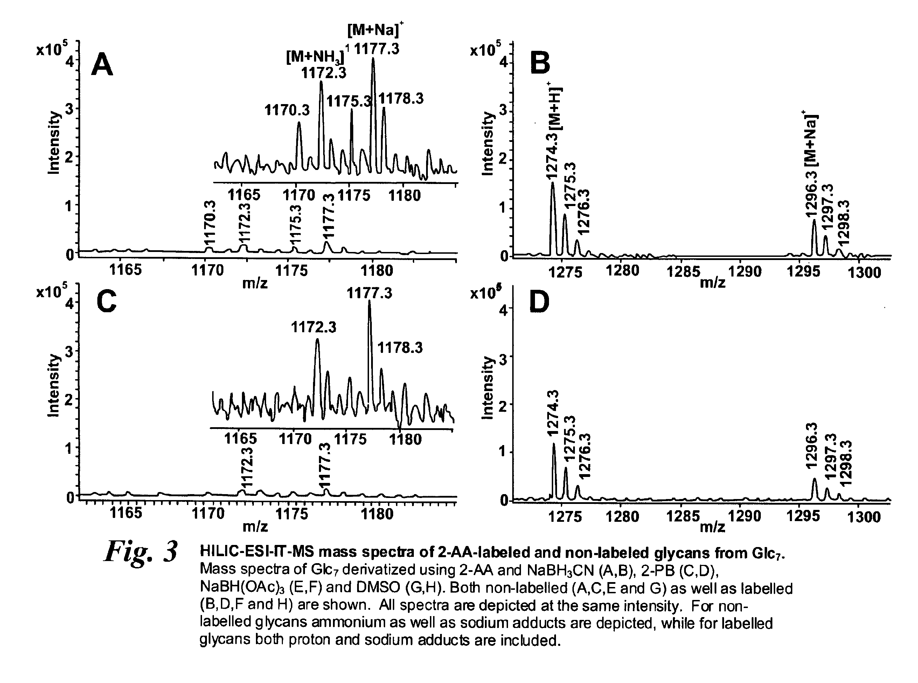

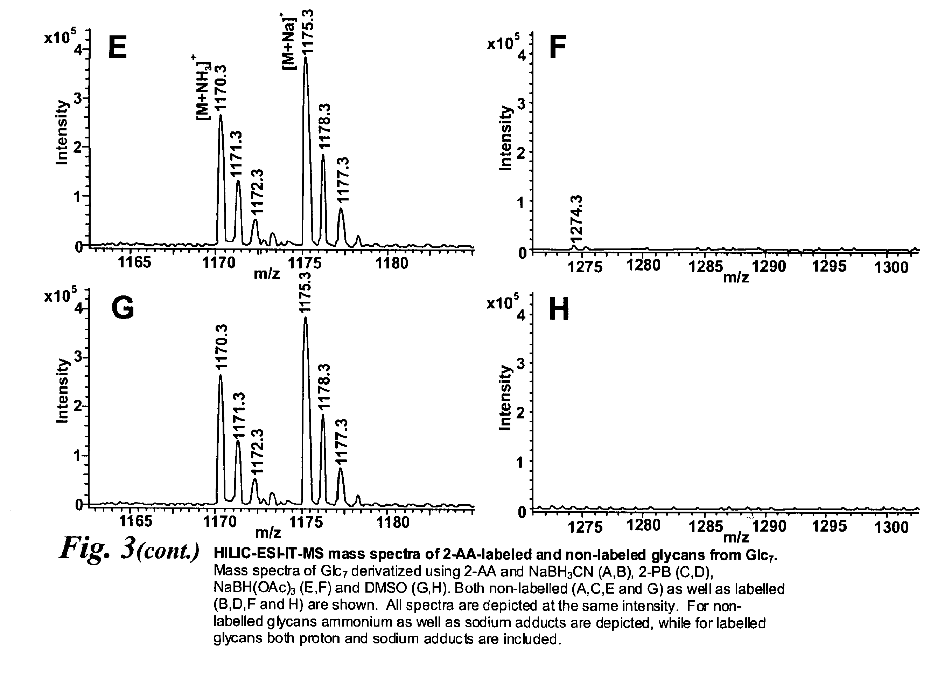

In order to determine labelling efficacies, samples were purified by graphitized carbon solid phase extraction and analyzed by HILIC-ESI-IT-MS, which allowed the parallel registration of labelled glucose oligomers, unlabelled species, as well as side products of the labelling reaction. As an example of the obtained data, mass spectra of the 2-AA-labeled Glc₇, as well as the remaining Glc₇ are depicted in Figure 3.

NaBH₃CN and 2-picoline-borane show nearly complete labelling, with high-intensity signals for the proton adducts (m/z 1274.3) as well as sodium adducts (m/z 1296.3) of the 2-AA labeled Glc₇ (Figure 3B and D), while only low amounts of unlabelled Glc₇ were detected (ammonium adducts at m/z 1170.3 and sodium adducts at m/z 1175.3 in Figure 3A and C). In contrast, hardly any labelled Glc₇ was observed for NaBH(OAc)₃ (Figure 3F), with most of the Glc₇ being present in unlabeled form (Figure 3E). In accordance with the HILIC-HPLC-FL data, the LC-MS data for labelled versus unlabeled Glc₄, Glc₅, Glc₆ and Glc₇, which are summarized in Table 1, indicate efficient labelling for both sodium cyanoborohydride and 2-picoline-borane, while NaBH(OAc)₃ appeared to be a very inefficient reducing agent.

N-glycans derived from human plasma were labelled using NaBH₃CN, 2-picoline-borane and NaBH(OAc)₃. HILIC-HPLC-FLU chromatograms as depicted in Figure 2D, showed similar labelling performance of NaBH₃CN and 2-picoline-borane. Again, NaBH(OAc)₃ appeared to be much less efficient.

### Example 2: labelling of N-glycans from human plasma with APTS (8-Aminopyrene-1,3,6-trisulfonic acid) using NaBH₃CN and 2-PB

### Materials

Dimethylsulphoxide (DMSO), Nonidet P-40 (NP-40), triethylamine (TEA), APTS, NaBH₃CN and 2-picoline-borane were obtained from Sigma-Aldrich (Zwijndrecht, The Netherlands). Sodium dodecyl sulphate (SDS) was bought from United States Biochemicals (Cleveland, OH). PNGaseF was obtained from Roche Diagnostics (Mannheim, Germany). Glacial acetic acid was from Merck (Darmstadt, Germany). Biogel P-10 was obtained from Bio-Rad (Veenendaal, The Netherlands), while citric acid and ethanol were from Merck (Darmstadt, Germany). Acetonitrile was purchased from Biosolve (Valkenswaard, The Netherlands).

### Preparation of oligosaccharides

N-glycans from human plasma were prepared as described in Ruhaak et al, Anal. Chem. 2008, 80, 6119-26, which is incorporated herein by reference. Proteins from 10 µl of plasma were denatured after addition of 20 µl 2% SDS by incubation at 60°C for 10 min. Subsequently, 10 µl 4% NP-40 and 0.5 mU of PNGase F in 10 µl 5x phosphate-buffered saline (PBS) was added to the samples. The samples were incubated over night at 37°C for N-glycan release.

### Labelling of oligosaccharides

2 µl of N-glycan solution were mixed with 2 µl of a freshly prepared solution of label (APTS; 20 mM in 3.6 M citric acid) in a v-bottom 96-wells plate. 2 µl aliquots of freshly prepared reducing agent solutions (different molarities of NaBH₃CN or 2-picoline borane in DMSO) were added, followed by 5 min of shaking and incubation at 37 °C for 16 hours. To stop the reaction, 50 µl of acetonitrile: water (80:20 v/v) were added and the samples were mixed for 5 min.

### HILIC-SPE

Free label and reducing agent were removed from the samples using HILIC-SPE. An amount of 100 µL of a 100 mg/mL Biogel P-10 suspension in water:EtOH:acetonitrile (70:20:10 v/v) was applied to each well of a 0.45 µm GHP filter plate (Pall Corporation, Ann Arbor, MI). Solvent was removed by application of vacuum using a vacuum manifold (Millipore, Billerica, MA). All wells were prewashed using 5 × 200 µL of water, followed by equilibration using 3 × 200 µL of ACN/water (80:20 v/v). The samples were loaded to the wells, and the plate shaken for 5 min on a shaker to enhance glycan binding. The wells were subsequently washed using 5 × 200 µL of ACN/water (80:20 v/v) containing 100 mM TEA, followed by 3 × 200 µL of ACN/water (80:20 v/v). The labelled N-glycans were eluted using subsequently 100 (to swell the biogel particles), 200 and 200 µL of water, and the eluates were collected in a 96-well V-bottom deep well plate.

### Capillary gel electrophoresis with laser-induced fluorescence (CGE-LIF) using ABI-3730 DNA sequencing equipment

Labelled N-glycans were analyzed using HILIC-HPLC with fluorescence detection. 60 µL of DMSO was dispensed in a PCR plate (Thermo Fischer Scientific via Westburg, Leusden, The Netherlands). 6 µL of N-glycan eluate was added to the DMSO, and the samples were analysed using an ABI-3730 DNA sequencer. The injection voltage was set at 7.5 kV, and the running voltage was 10 kV. The system was equipped with a 48 channel array with capillaries of 50 cm, and the capillaries were filled with POP-7 buffer (Applied Biosystems). The running buffer was purchased from Applied Biosystems.

### Data processing

Datafiles were converted to xml files and then loaded into Matlab version 2007a (The Mathworks, Inc., Natick, MA). After background subtraction, peak heights were determined for the 15 most abundant peaks.

### Results

For glycan analysis by CE-LIF or CE-MS, derivatization of glycans with the APTS label is favourable since it has three sulfonic acids, which generate three negative charges. Recently, a method was developed for fast profiling of N-glycan patterns with the aid of a DNA-sequencer (Laroy W, Contreras R, Callewaert N (2006) Glycome mapping on DNA sequencing equipment, Nat Protoc 1: 397-405, which is incorporated herein by reference). Using this application, we can currently screen 48 samples in one run of about 1 hour.

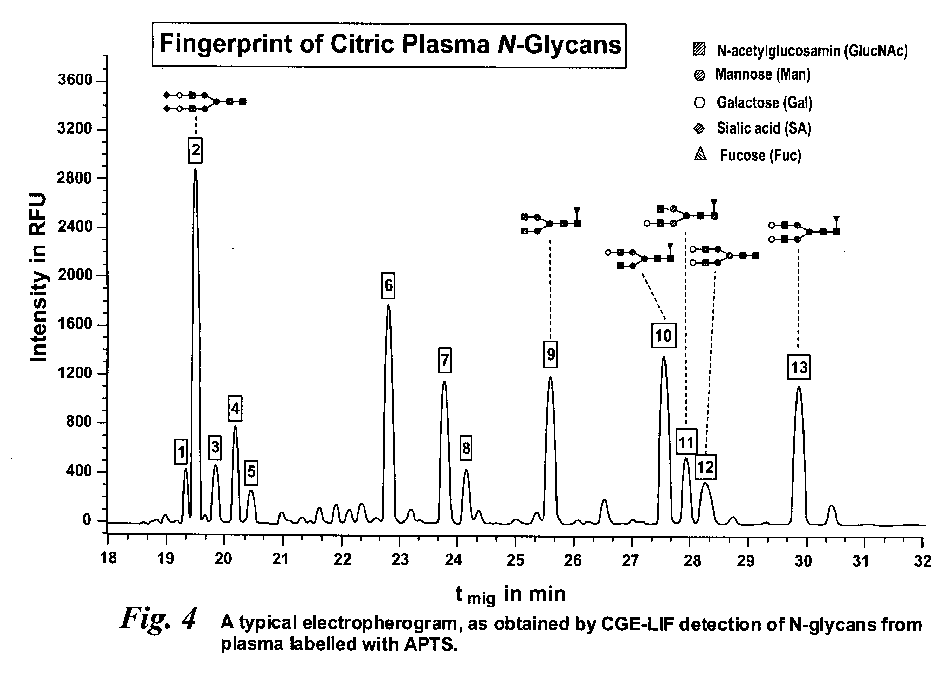

Plasma N-glycans were analyzed in APTS-labelled form using a DNA-sequencer. A characteristic electropherogram with peak number annotations and some glycan structures is included in Figure 4.

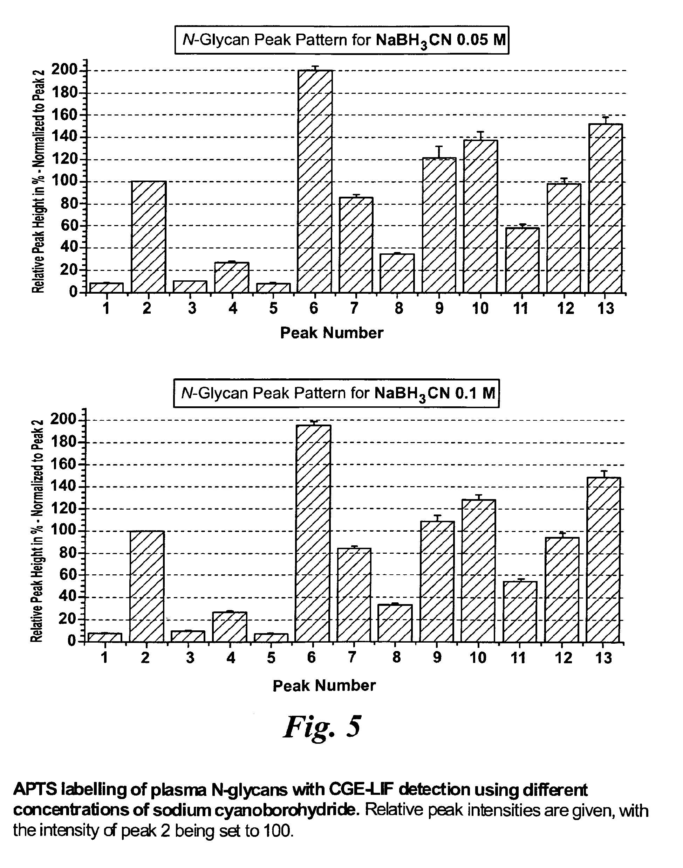

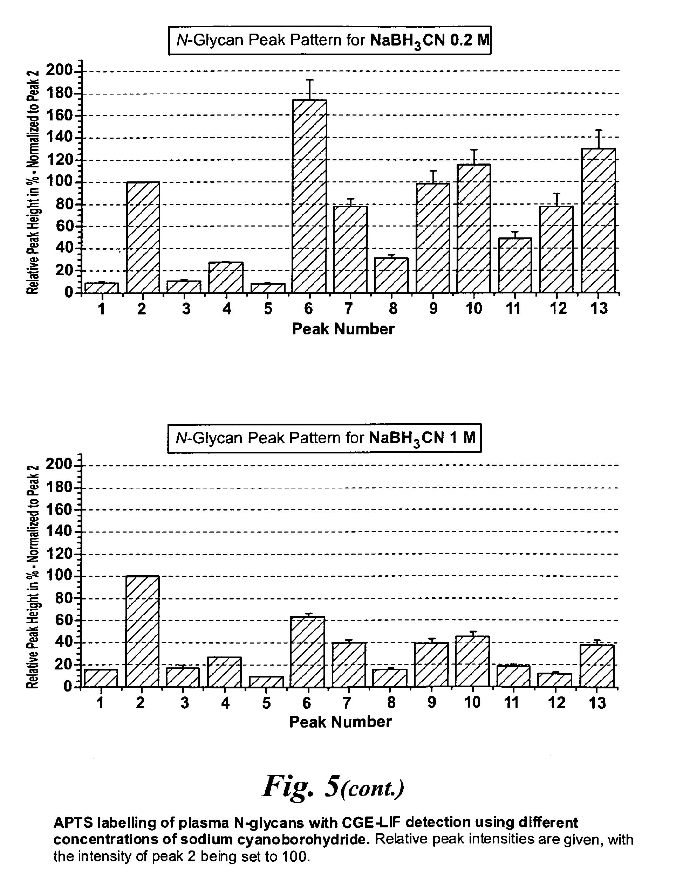

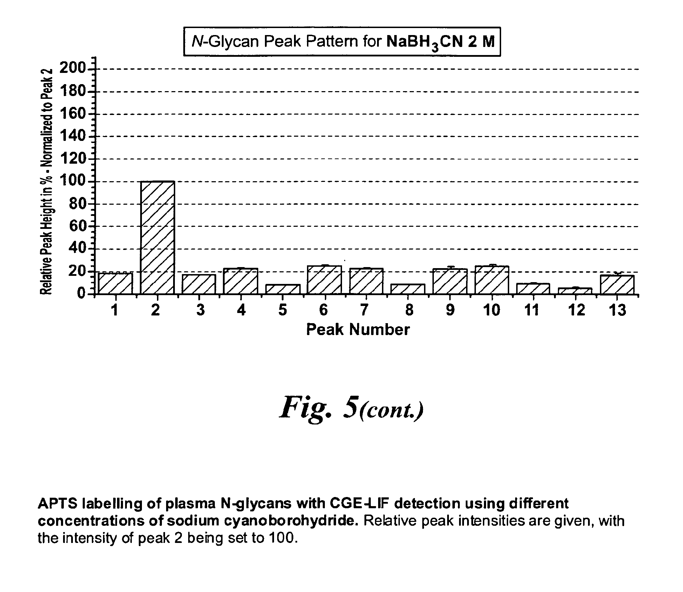

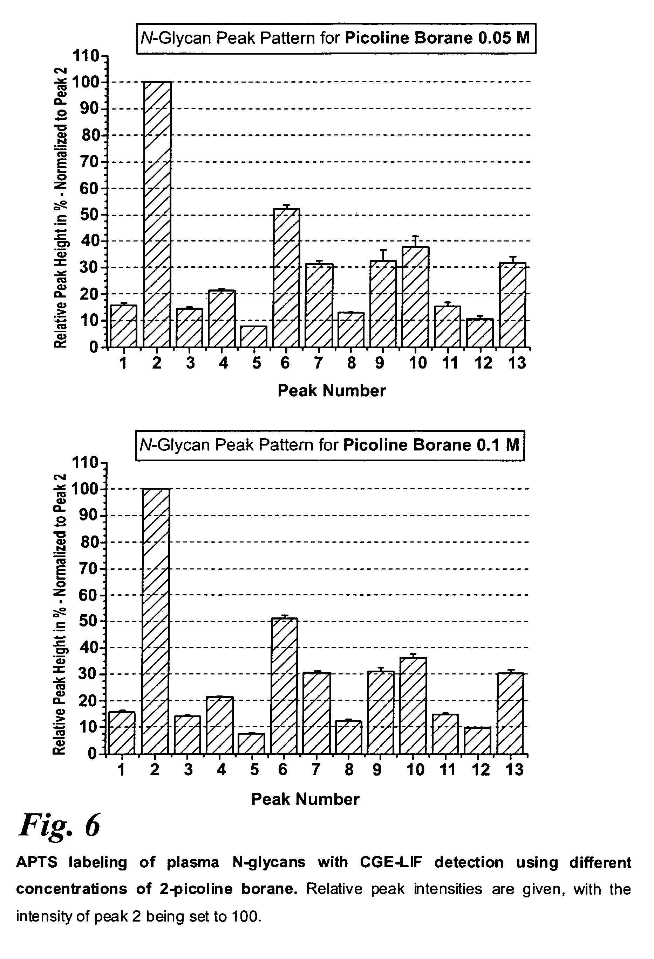

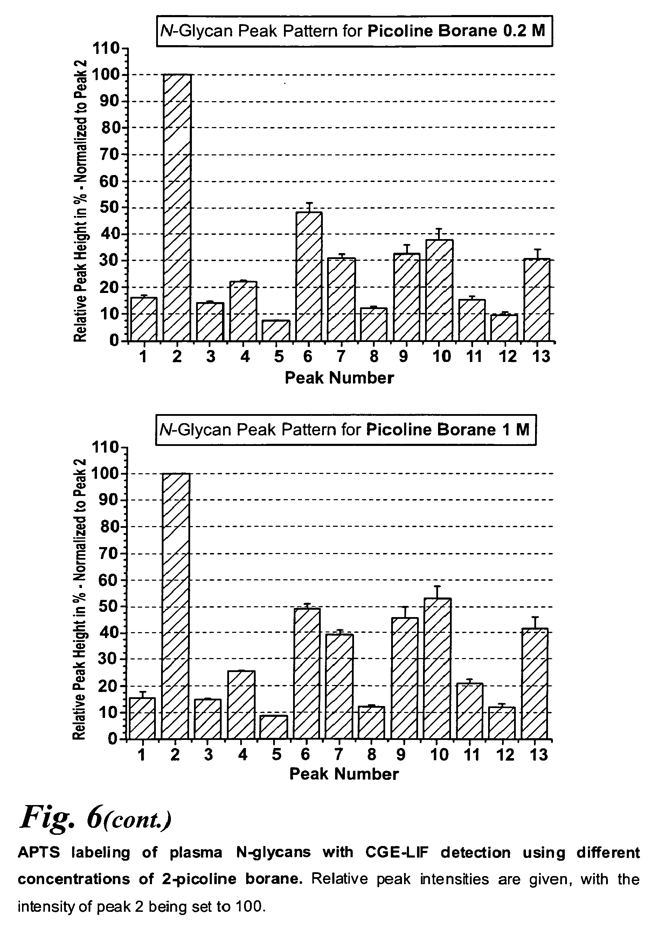

2-Picoline borane and NaBH₃CN were compared as to their efficacy in reductive amination labelling of plasma N-glycans with APTS. APTS-labelled N-glycan profiles as well as yields obtained with five different concentrations of NaBH₃CN (2M, 1 M, 0.2M, 0.1M and 0.05M) and four different concentrations of 2-picoline borane (1 M, 0.2M, 0.1 M and 0.05M) were compared, and the intensities of 13 peaks migrating at defined positions, were determined. Figure 5 shows the relative intensities of the 13 peaks with varying concentrations of sodium cyanoborohydride. Notably, the profiles showed a pronounced variation with increasing concentrations of reducing agent. In contrast, similar experiments performed with 2-picoline borane showed only little variation of APTS-glycan profiles with different concentrations of reducing agent (Figure 6).

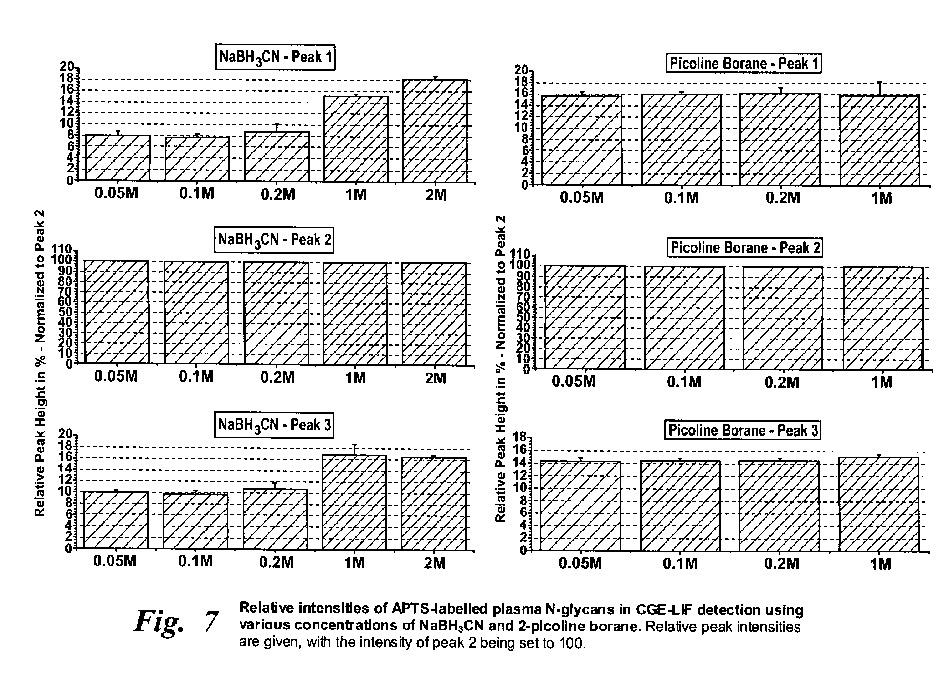

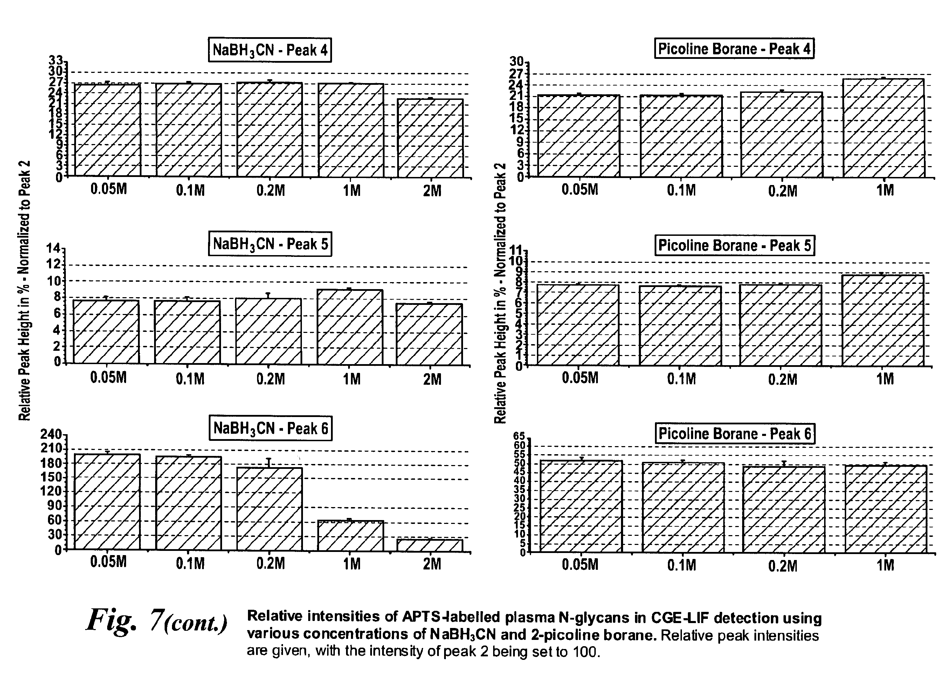

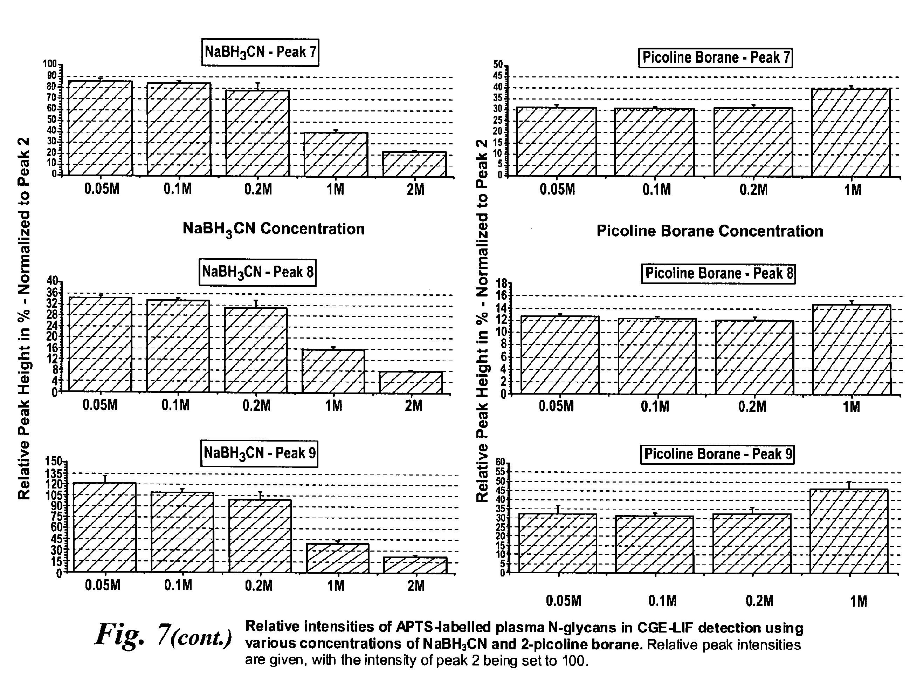

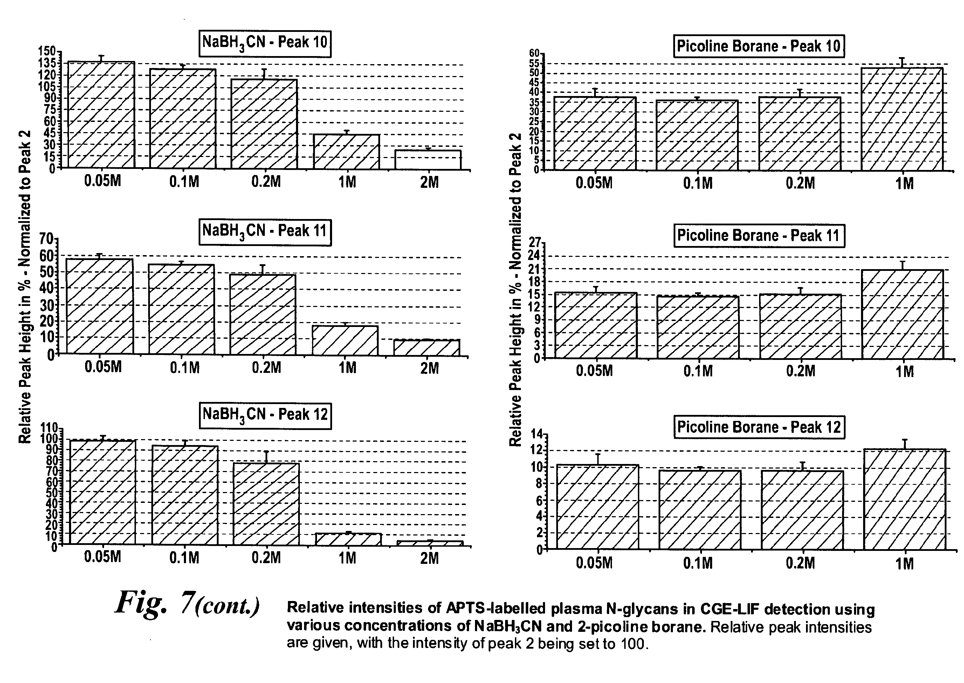

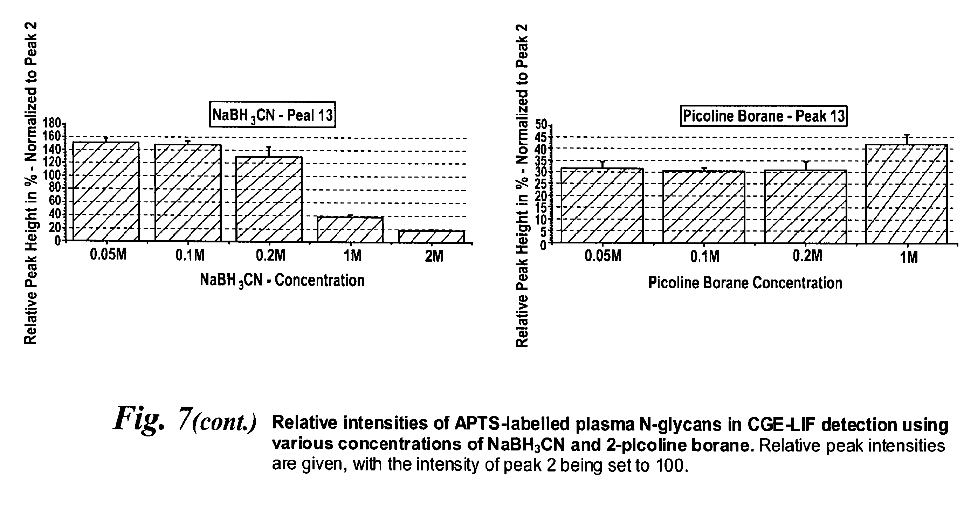

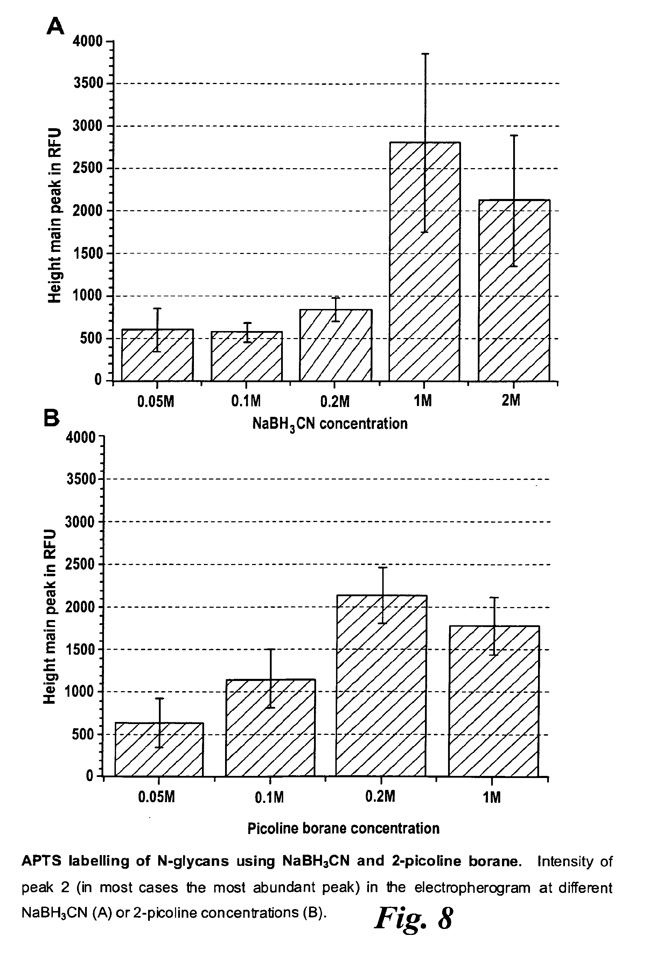

In order to clarify the variation of the relative intensities of individual peaks with varying concentrations of reducing agents, additional graphs of the same data were generated (Figure 7). For both sodium cyanoborohydride (left column) and 2-picoline borane (right column), the relative intensities of individual peaks are depicted with varying concentrations of reducing agent. Peaks 1 and 3 showed an increasing relative abundance with increasing concentrations of sodium cyanoborohydride. Peaks 6, 7, 8, 9, 10, 11, 12, and 13 showed a decreasing relative intensity with increasing concentrations of sodium cyanoborohydride. In contrast, the data obtained for 2-picoline borane (right column of Figure 8) showed hardly any dependence of relative peak intensities on the concentration of reducing agent.

As well as comparing the robustness of the obtained profiles (relative labelling efficacies), the absolute labelling efficacies obtained for peak 2 with varying concentrations of the two reducing agents were also compared (Figure 8). The biggest differences were observed for concentrations of 0.1 M and 0.2 M of reducing agents. At reducing agent concentrations of 0.1 M, the yield of peak 2 obtained with 2-picoline borane was twice as high as the yield obtained with sodium cyanoborohydride. At reducing agent concentrations of 0.2 M, the difference in efficacy was even higher.

In conclusion, Example 2 shows that 2-picoline borane is more efficient in labelling N-glycans with APTS than sodium cyanoborohydride. Moreover, the obtained profiles of APTS-labelled N-glycans are much more robust with 2-picoline borane than with sodium cyanoborohydride. At sub-optimal concentrations of 0.1 M and 0.2 M, sodium cyanoborohydride seemed to produce fast-migrating ATPS-labelled N-glycan species (peaks 1 to 3) less efficiently than the late-eluting species (peaks 6 to 13). In the case of sodium cyanoborohydride, labelling efficacy seems to be influenced by the charge of the N-glycans: fast-migrating peaks, which are known to correspond to sialylated N-glycans, are labelled rather inefficiently at lower concentrations of this reducing agent, whilst the slow-migrating species (peaks 6-13), which correspond to neutral N-glycans, are less prone to incomplete labelling with lowered concentrations of sodium cyanoborohydride. Again, no such dependence of labelling efficacies on structural features such as charge has been observed for 2-picoline borane.

The invention is defined by the following claims.

## Claims

1. Use of 2-picoline borane (2-PB) for the reductive amination of a carbohydrate.

2. A use according to claim 1 wherein the carbohydrate is reacted with a labelling agent in the presence of 2-PB.

3. A process for labelling a carbohydrate, the process comprising contacting the carbohydrate with a labelling agent in the presence of 2-picoline borane (2-PB) to produce a labelled carbohydrate.

4. A use or process according to claim 2 or 3 wherein the labelling agent is an amine-substituted chromophore of fluorophore.

5. A use or process according to claim 4 wherein the amine-substituted chromophore of fluorophore is selected from 2-aminobenzoic acid (2-AA), 2-aminobenzamide (2-AB), 8-aminopyrene-1,3,6-trisulfonic acid (APTS), 2-aminopyridine (2-AP), [D₆]-PA, [D₄]-2-AA, 2,5-diaminopyridine and 2-amino-6-amidobiotinylpyridine (BAP).

6. A use or process according to claim 4 wherein the amine-substituted chromophore of fluorophore is selected from 2-aminobenzoic acid 2-AA, 2-AB and APTS.

7. A use or process according to any of the preceding claims wherein the carbohydrate is derived from human, animal or plant origin, or from a recombinantly expressed or biotechnologically expressed protein.

8. A use or process according to claim 7 wherein the carbohydrate is prepared by full or partial hydrolysis or degradation of a polysaccharide.

9. A use of process according to claim 7 wherein the carbohydrate is prepared by full or partial chemical synthesis.

10. A use or process according to claim 7 wherein the carbohydrate is a glycan derived from a glycoprotein or a glycolipid.

11. A method for detecting a carbohydrate in a sample, the method comprising:
(i) contacting the carbohydrate in the sample with a labelling agent in the presence of 2-picoline borane (2-PB) to produce a labelled carbohydrate as defined in any of claims 3 to 9; and
(ii) detecting the labelled carbohydrate.

12. A method according to claim 11 wherein the detecting step comprises liquid chromatography coupled to fluorescence detection.

13. A method according to claim 12 wherein the liquid chromatography coupled to fluorescence detection is hydrophilic interaction chromatography with fluorescence detection (HILIC-HPLC-FL).

14. A use, process or method according to any of the preceding claims, which is carried out simultaneously on a plurality of samples potentially containing a carbohydrate.

15. Any novel process, use or method generally as herein described, optionally with reference to the examples.
